(19) Europäisches Patentamt
European Patent Office
Office européen des brevets

(11) Veröffentlichungsnummer: **0 302 466 B1**

# (12) EUROPÄISCHE PATENTSCHRIFT

(45) Veröffentlichungstag der Patentschrift: **21.11.91**

(51) Int. Cl.⁵: **A61K 47/22**

(21) Anmeldenummer: **88112611.4**

(22) Anmeldetag: **03.08.88**

## (54) Cyclopeptide als Resorptionsförderer bei Applikation auf die Schleimhäute.

(30) Priorität: **07.08.87 DE 3726324**

(43) Veröffentlichungstag der Anmeldung:
**08.02.89 Patentblatt 89/06**

(45) Bekanntmachung des Hinweises auf die Patenterteilung:
**21.11.91 Patentblatt 91/47**

(84) Benannte Vertragsstaaten:
**AT BE CH DE ES FR GB GR IT LI LU NL SE**

(56) Entgegenhaltungen:
**EP-A- 0 159 167**
**DE-A- 3 532 074**
**GB-A- 2 095 994**

**CHEMICAL ABSTRACTS, Band 97, 1982, Seite 400, Zusammenfassung Nr. 98358y, Columbus, Ohio, US; & JP-A-82 80 314 (KYOTO PHARMACEUTICAL INDUSTRIES, LTD) 19-05-1982**

(73) Patentinhaber: **HOECHST AKTIENGESELLSCHAFT**
**Postfach 80 03 20**
**W-6230 Frankfurt am Main 80(DE)**

(72) Erfinder: **Sandow, Jürgen Kurt, Dr.**
**Am Haideplacken 22**
**W-6240 Königstein/Taunus(DE)**
Erfinder: **Schmiedel, Rainer, Dr.**
**Gundelhardtstrasse 2**
**W-6233 Kelkheim (Taunus)(DE)**
Erfinder: **Wirth, Klaus, Dr.**
**Johannesallee 28**
**W-6230 Frankfurt am Main 80(DE)**
Erfinder: **Merkle, Hans Peter, Prof. Dr.**
**Hartmann-Ibach-Strasse 70**
**W-6000 Frankfurt am Main(DE)**
Erfinder: **Raehs, Susanne**
**Habsburger Allee 26**
**W-6000 Frankfurt a.M. 60.(DE)**

EP 0 302 466 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Hilfsstoffe, insbesondere Cyclopeptide, zur Förderung der Resorption von Peptiden und Proteinen bei Applikation auf die Schleimhäute.

Die Anwendung von Peptiden und Proteinen als Arzneimittel ist erheblich erschwert durch die Probleme einer geeigneten galenischen Zubereitung, aus der das therapeutisch oder diagnostisch anzuwendende Peptid oder Protein in ausreichender Menge und zuverlässig resorbiert wird.

Eine dem Stand der Technik entsprechende Lösung ist die Verabfolgung einer oder mehrerer täglicher Einzeldosen durch nasale Applikation, sei es in Form von Nasentropfen oder durch Einsprühen einer geeigneten Lösung in die Nase (J. Sandow, W. Petri in Transnasal Systemic Medications, Verlag Elsevier, (1985) 183-199). Es ist bekannt, hierzu gut verträgliche wäßrige Lösungen mit Zusatz von Konservierungsstoffen zu verwenden. Die bekannten Hilfsstoffe zur Erhöhung der Resorption (absorption enhancers) sind sämtlich schleimhautreizend oder durch unangenehmen Geruch bzw. Geschmack ungeeignet und führen oft bereits bei einmaliger Applikation zu erheblichem Schmerz und Tränenfluß oder erzeugen bei mehrfacher Applikation eine fortschreitende Reizung und Entzündung der Nasenschleimhäute. Dies gilt z.B. für Derivate der Fusidinsäure, für Gallensäuren und verschiedene Glykole (Polyethylenglykol, Propylenglykol).

In GB-A 2 095 994 werden N-Acylpeptide oder N-Acylaminosäure Derivate beschrieben, die als Absorptionsförderer in peptid-oder proteinhaltigen pharmazeutischen Zubereitungen enthalten sind.

Der Erfindung liegt die Aufgabe zugrunde, Hilfsstoffe zu finden, die die Resorption bei Applikation auf die Schleimhäute fördern und gut verträglich, d.h. nicht schleimhautreizend sind.

Diese Aufgabe wird erfindungsgemäß dadurch gelöst, daß Hilfsstoffe der allgemeinen Formel I

$$\text{cyclo-(B-A/B-X-NH}(CH_2)_{2-4}\text{-}\underset{\underset{\displaystyle R}{|}}{\underset{\displaystyle NH}{\underset{|}{CH}}}\text{-CO-B-L-L)} \qquad (I)$$

worin

- B Lysin, Ornithin, Histidin, 2,4-Diaminobuttersäure oder Arginin,
- A/B Lysin, Ornithin, Histidin, 2,4-Diaminobuttersäure, Arginin, Asparaginsäure oder Glutaminsäure,
- X Asparagin, Glutamin, Serin oder Threonin,
- L Leucin, Isoleucin, Valin, Threonin, Phenylalanin oder Tryptophan und
- R Wasserstoff oder ein Acylrest mit folgenden Strukturen

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

wobei die Aminosäuren jeweils in der D- oder L-Form vorliegen können,
bedeuten, sowie deren physiologisch verträgliche Salze zur Förderung der Resorption von Peptiden und Proteinen bei Applikation auf die Schleimhäute verwendet werden.

Ac steht hier für

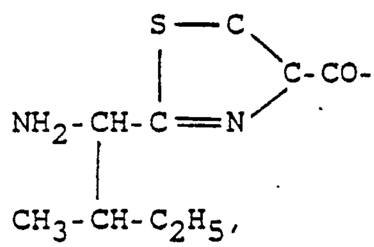

wobei S auch als Sulfoxid oder Sulfon vorliegen und die Doppelbindung hydriert sein kann,

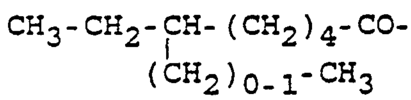

oder

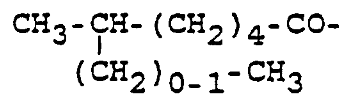

.

Bevorzugt sind solche Reste B, A/B, X und L, die sich von natürlich vorkommenden Aminosäuren (s. z.B. Schröder, Lübke, The Peptides, Volume I, New York 1965) und 2,4-Diaminobuttersäure, deren Antipoden und einfachen Metaboliten ableiten, die, falls chiral, in der D- oder L-Form vorliegen können.

Falls nicht anders angegeben, werden die Dreibuchstabensymbole (vgl. z.B. Pure & Appl. Chem. 56 (1984) 595-624 und Eur. J. Biochem. 138 (1984) 9-37) für die Reste der Aminosäuren verwendet. Diesen Symbolen wird das Symbol "D" vorangestellt, wenn es sich um den Rest einer D-Aminosäure handelt; Reste ohne Konfigurationssymbol sind L-konfiguriert.

Als besonders geeignet erwiesen sich die als Peptidantibiotika bekannten Bacitracine, Colistine, Circuline und Polymyxine [R. Reiner, Antibiotica und ausgewählte Chemotherapeutica, Georg Thieme Verlag Stuttgart], wie z.B.

Bacitracin A:

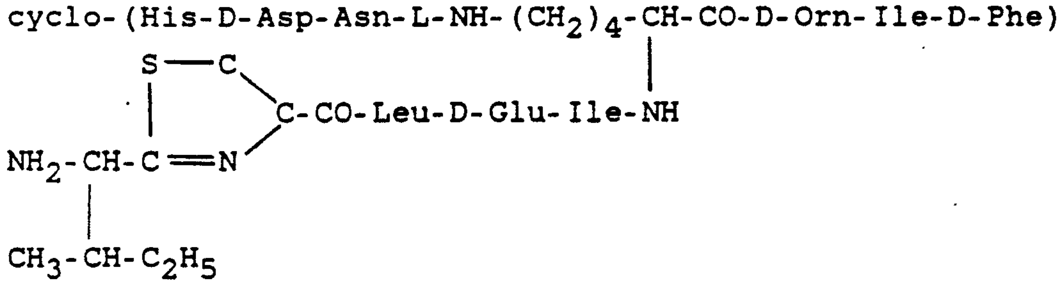

Colistin A oder B:

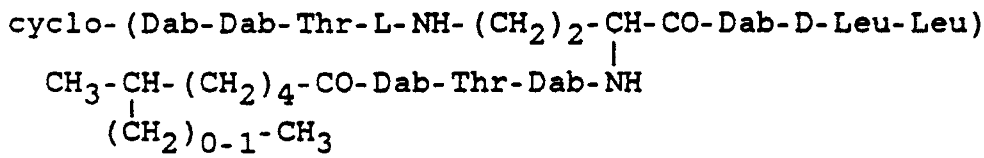

Circulin A oder B:

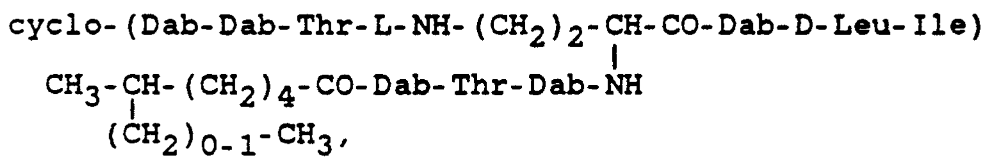

Polymyxin $B_1$ oder $B_2$:

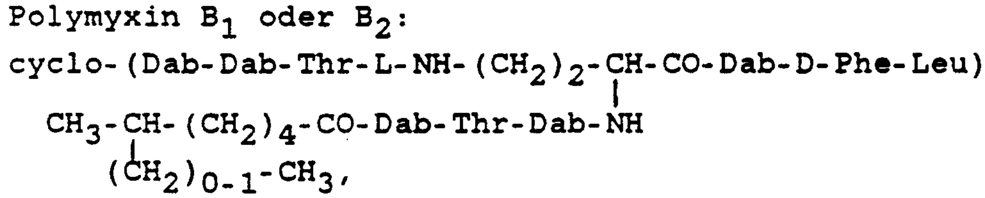

Polymyxin $D_1$ oder $D_2$:

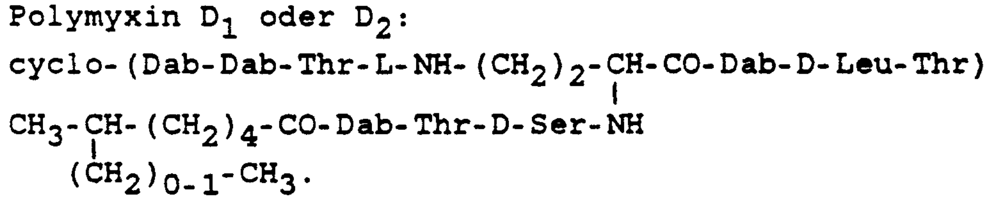

Wegen der reduzierten antibiotischen Wirkung und Toxizität sind auch die Cyclopeptide der allgemeinen Formel I mit teilweiser oder völlig abgebauter Seitenkette von Interesse [T. Suzuki et al., J. Biochem. Tokyo 54 (1963) 555; 56 (1964) 335; S.T. Chihara et al., Agr. Biol. Chem. 37 (1973) 2455-2463; M. Vaara und T. Vaara, Antimicrobial Agents and Chemotherapy 24 (1983) 107-113], wie z.B. das Polymyxin B Nonapeptid (PMBN) oder das Polymyxin B Heptapeptid (PMBH).

Polymyxin B Nonapeptid (PMBN):

cyclo-(Dab-Dab-Thr-L-NH-$(CH_2)_2$-CH-CO-Dab-D-Phe-Leu)
|
H-Thr-Dab-NH

Polymyxin B Heptapeptid (PMBH):

cyclo-(Dab-Dab-Thr-L-NH-$(CH_2)_2$-CH-CO-Dab-D-Phe-Leu)
|
$NH_2$

Die erfindungsgemäßen Verbindungen können unter Benutzung der allgemeinen Methoden der Peptidchemie (Houben-Weyl, Methoden der organischen Chemie, 15/1 und 2), beispielsweise stufenweise vom C-terminalen Ende oder durch Segment-Kondensation und anschließende Cyclisierung, wie z.B. in EP-A 87 106 224.6 beschrieben oder durch Isolierung und anschließenden Abbau - gegebenenfalls durch enzymatische Spaltung - der entsprechenden natürlich vorkommenden Peptide [T. Suzuki et al., J. Biochem. Tokyo 54 (1963) 555; 56 (1964) 335; S. T. Chihara et al., Agr. Biol. Chem. 37 (1973) 2455-2463; M. Vaara und T. Vaara, Antimicrobial Agents and Chemotherapy 24 (1983) 107-113] hergestellt werden.

Die erfindungsgemäßen Verbindungen tragen in ganz erheblichem Maße zur Verbesserung der Resorption von Peptiden und Proteinen bei Applikation auf die Schleimhäute bei. So beträgt die Steigerung der Wirksamkeit der Peptide oder Proteine nach dem Zusatz der erfindungsgemäßen Verbindungen 300 bis 400 % und kann im Einzelfall mehr als 1000 % betragen. Eine Wirksamkeitssteigerung wurde z.B. nachgewiesen für LHRH (Gonadoliberin), LHRH Agonisten (Buserelin und ähnliche Nona- und Decapeptide), Growth Hormone Releasing Hormone und Agonisten, ACTH (Corticotropin) und Agonisten sowie Calcitonin und Agonisten.

**Beispiele**

1. Die Wirkung von Cyclopeptiden auf die nasale und rektale Resorption von LHRH(Gonadorelin) und LHRH Agonisten (z.B. Buserelin) wurde in folgender Weise untersucht: Weibliche Ratten von 60 g Körpergewicht werden mit Pferdeserumgonadotropin (PMSG) 10 I.E. vorbehandelt (Tag 1, 09:00) zur Follikelstimulierung. Am dritten Tag nach der Vorbehandlung wird die spontan gegen 14:00 auftretende Ovulation unterdrückt durch Injektion von Phenobarbital (4 mg/Tier i.p. um 13:00). Die Behandlung mit der Testsubstanz (z.B. LHRH oder Buserelin intranasal in einer physiologisch verträglichen Pufferlösung mit pH 3,0-7,5 in einem Volumen von 2-20 $\mu$1, rektale Applikation von Suppositorien etc.) erfolgt um 13:30. Diese Behandlung löst dosisabhängig die Ovulation aus. Am folgenden Versuchstag (Tag 4) werden die Tiere um 09:00 getötet, beide Eileiter präpariert und nach Anfärben mit Patentblau unter einem Stereomikroskop die Anzahl der Eizellen ausgezählt (Ovulationswirkung der Testsubstanzen). Die Erhöhung der Resorption der Testsubstanzen von Schleimhautoberflächen wird durch Vergleich der Wirkung auf die Ovulation in Abwesenheit oder nach Zusatz von z.B. Bacitracin 0,001-0,05 M beurteilt. In der gleichen Versuchsanordnung kann auch der dosisabhängige Anstieg von Luteinisierendem Hormon (LH) im Serum eine Stunde nach Behandlung als Parameter der Wirkung verwendet werden.
[Sandow J, von Rechenberg W & Jerzabek G (1976): The effect of LHRH, prostaglandins and synthetic analogues of LHRH on ovarian metabolism. Europ. J. Obstetr. Gynec. Reprod. Biol. 6, 185-190.]

Es zeigte sich, daß die ovulatorische Wirkung von 8 $\mu$g LHRH in physiologischer Kochsalzlösung mit 0,1 % Gelatine in dieser Versuchsanordnung die gleiche ist wie die Wirkung von 2 $\mu$g LHRH in einer Lösung von 0,001 M Bacitracin. Weiterhin zeigte sich dieser Effekt auch in Anwesenheit von 0,001 M Colistin, bei Untersuchung der LH-Freisetzung. Ebenso entsprach die ovulatorische Wirkung von 80 ng Buserelin in physiologischer Kochsalzlösung mit 0,1 % Gelatine in dieser Versuchsanordnung der Wirkung von 20 ng Buserelin in einer Lösung von 0,001 M Bacitracin. Die Verstärkung der Resorption zeigte sich auch anhand der Bestimmung der LH-Freisetzung durch Buserelin in Anwesenheit von 0,001 M Bacitracin. Hier fand sich eine Verstärkung um mehr als das Dreifache.

2. Eine andere zum Nachweis der Wirkungsverstärkung durch erhöhte Absorption geeignete Methode ist z.B. die Bestimmung der LH-Freisetzung bei männlichen Ratten (100 g Körpergewicht in Urethannarkose). Hier wird die Hormonfreisetzung über einen Zeitraum von 6-7 Stunden nach Behandlung (z.B. durch nasale oder rektale Applikation der Testsubstanzen in physiologischer Kochsalzlösung mit oder ohne Zusatz von Bacitracin, Polymyxin B oder ähnlichen Cyclopeptiden) verglichen. Es zeigte sich, daß

sowohl Bacitracin als auch Colistin und Polymyxin B (0,01 M) die Wirkung einer Dosis von 10 ng Buserelin um das 3,3 - 14-fache erhöhten, gemessen an den Flächen unter der Kurve.

Bei der rektalen Behandlung von Ratten mit Buserelin-Suppositorien im gleichen Modell trat die Ovulation ohne Zusatz von Bacitracin bei einer Dosis von 400 ng ein, während nach Zusatz von Bacitracin bereits bei einer Dosis von 50 ng Buserelin eine Ovulation ausgelöst wurde.

3. Die Wirkung von Cyclopeptiden auf die nasale Resorption von GHRH-Analogen wurde auf folgende Weise untersucht:

In dem gleichen Modell wie für die Ovulationswirkung von LHRH beschrieben kann am dritten Tag nach Vorbehandlung mit PMSG eine Prüfung der Freisetzung von Wachstumshormon (Growth Hormone, GH) durchgeführt werden. Die Bestimmung von GH im Serum erfolgt 15-120 Minuten nach Behandlung (z.B. nasal oder rektal), durch spezifischen Radioimmunoassay.

Es zeigte sich, daß die Wirkung von (DAla2) GRF29-amid ohne Bacitracin bei einer Dosis von 40 µg i.n. ohne Bacitracin der Wirkung einer Dosis von 20 µg mit Zusatz von 0,001 M Bacitracin entsprach. Der gleiche Befund wurde mit anderen GHRH-Agonisten erhoben, z.B. mit (DAla2 , NLeu27) GRF-29-amid und dem gentechnologisch hergestellten GHRH-Derivat (Leu27, Gly45) GHRH (1-44).

4. Die Wirkung von Cyclopeptiden auf die Resorption von ACTH (Corticotropin) und ACTH-Analogen wurde untersucht an männlichen Ratten (100 g Körpergewicht) nach Behandlung unter Narkose mit Pentobarbital oder Ether. Als Parameter der Wirkung wurde die Corticosteron-Freisetzung im Serum durch spezifischen Radioimmunoassay bestimmt. Es zeigte sich, daß nach nasaler Behandlung z.B. mit dem ACTH-Analogen Alsactide (ACTH-17) in einer Dosis von 5 µg in Gegenwart von 0,01 M Bacitracin die Corticosteron-Freisetzung über 3 h um das 5-fache gesteigert war.

5. Die Wirkung von Cyclopeptiden auf die Resorption von Calcitonin und Calcitonin-Analogen (z.B Salmon-Calcitonin) kann untersucht werden an männlichen Ratten von 100 oder 200 g Körpergewicht, z.B. nach intranasaler Behandlung mit Salmon-Calcitonin, durch Bestimmung der Serum-Calcium-Konzentration über einen Zeitraum von 1-6 h nach der Behandlung. Hier wurde gefunden, daß die Wirkung von 0,6-1,2 µg Salmon-Calcitonin durch den Zusatz z.B. von 0,01 M Bacitracin um das 4- 6-fache gesteigert wurde.

6. Die Verträglichkeit verschiedener Cyclopeptide, wie sie als Hilfsstoffe für die Erhöhung der Resorption verwendet werden, kann an der isolierten Magenschleimhaut des Meerschweinchens geprüft werden. [Wirth K, Bickel M & Deutschländer N (1987): Patent blue permeation through the isolated guinea pig gastric mucosa: a quantitative method for the assessment of gastric irritants. Med. Sci. Res. 15, 881.]

Hier zeigte sich, daß durch Gallensäuren, z.B. die stark resorptionssteigernde Desoxycholsäure bereits in einer Konzentration von 0,002 M eine Schleimhautschädigung verursacht wird, die zu gesteigerter Permeation von Patentblau führt, während für die Resorptionssteigerung an der Ratte, z.B. geprüft mit dem LHRH-Analogen Buserelin eine 10-fach höhere Konzentration von 0,02 M erforderlich ist. Es kann dagegen eine Konzentration von mehr als 0,006 M Bacitracin der Schleimhaut ohne Schaden zugesetzt werden, während bereits eine Zugabe von 0,001 M Bacitracin zu einer 3-4-fachen Resorptionserhöhung z.B. von LHRH-Agonisten, GHRH-Agonisten und ähnlichen Wirkstoffen führt.

Ferner verursachen die erfindungsgemäßen Verbindungen beim Menschen keine Schmerzempfindung bei nasaler Applikation von 1 bis 200 µl einer Konzentration von $10^{-5}$ bis $10^{-1}$ mol/l. Sie führen wie in Beispiel 6 gezeigt, am Modell der isolierten Magenschleimhaut nicht zu einer Schädigung. Die lokale Anwendung der gleichen Konzentration durch vaginale, rektale oder buccale Arzneiformen (d.h. z.B. Filme, Tabletten, Suppositorien) führt ebenfalls zu keiner Schleimhautreizung. Bei rektaler Applikation von Harzfettsuppositorium mit einem Wirkstoffgehalt von 1 mg Bacitracin bei Ratten von 100 g Körpergewicht zeigte sich 3 Stunden nach Behandlung keine entzündliche Veränderung der Rektalschleimhaut. Die resorptionsfördernde Wirkung der erfindungsgemaßen Verbindungen wurde auch in einem Prüfmodell, wie z.B. in "Transnasal Systemic Medications", (Hrsg. Y. W. Chien, Verlag Elsevier, 1985) beschrieben, an Ratten und auch an Menschen nachgewiesen.

Für die meisten der heute bekannten Peptide und Proteine, die als Therapeutika oder Diagnostika verwendet werden oder in nächster Zeit zur Anwendung kommen werden, ist eine Applikation auf die Schleimhäute, wie z.B. die nasale, buccale, rektale oder vaginale Anwendung, insbesondere jedoch die nasale, zweckvoll und möglich.

Hierfür eignen sich Peptide und Proteine, die aus 3 bis 225 Aminosäuren bestehen, wie z.B. TRH (Protirelin, Thyroliberin), LHRH (Gonadoliberin), chemisch modifizierte Analogpeptide der hypothalamischen regulatorischen Hormone wie z.B. Buserelin, Somatostatin und cyclische Somatostatinanaloga, Somatorelin (GRH)-Analoga, Analogpeptide von Hypophysenhormonen wie z.B. das Corticotropin-Analogon Alsactide (ACTH-17), Calciumregulierende Hormone (Calcitonin, Parathormon) und ihre Analoga sowie gastrointestinale Hormone (z.B. Secretin und Cholecystokinin) und pankreatische Hormone (Insulin und Insulin-Analoga).

Besonders geeignet sind solche mit 3 bis 51 Aminosäuren.

Insbesondere seien genannt:

| Name des Peptids oder Proteins | Verwendung z.B. bei: | Zahl der Aminosäuren |
|---|---|---|
| Oxytocin | Wehenschwäche | 9 |
| Vasopressin | Diabetes insipidus | 9 |
| Ornipressin | Blutungen | 9 |
| Desmopressin | Diabetes insipidus | 9 |
| Corticotropin (ACTH) | entzündlichen Erkrankungen | 39 |
| Tetracosactid | entzündliche Erkrankungen | 24 |
| Alsactid | " | 17 |
| Insulin | Diabetes mellitus | 51 |
| δ-Schlaf ind. Peptid | Schlafstörungen | 9 |
| Secretin | Magenblutungen | 27 |
| Cholecystokinin | Erkrankungen der Gallenwege, als Appetitzügler | 8-32 |
| Somatoliberin (GRH) | Minderwuchs | 44 |
| [D-Ala$^2$]Somatoliberin-(1-29)-amid | " | 29 |
| Somatoliberinyl-glycin | " | 45 |
| Glucagon | Hypoglykämie | 29 |
| Somatostatin | Magenblutungen | 14 |
| Octreotide | Tumoren | 8 |
| Spantide | Substanz P-Hemmung | 11 |
| Corticoliberin (CRF) | Hypophysendiagnostikum | 41 |
| Bradykinin-Antagonisten | Schmerz, Schnupfen | 9-11 |
| Atriopeptin III | Herz- und Nieren-Insuffizienz | 24 |
| ANF- (99-126) | " | 28 |
| Thymopentin | rheumatische Arthritis | 5 |
| Interferon-α | Erkältung | 125 |
| Thyroliberin (TRH) | Hypophysendiagnostikum | 3 |
| Gonadoliberin (LHRH) | Kryptorchismus, Sterilität | 10 |
| Buserelin | Prostata-Krebs, Endometriose | 9 |
| Goserelin | " | 10 |
| Triptorelin | " | 10 |
| LH-RH-T | " | 9 |
| Leuprorelin | " | 9 |
| Lutrelin | " | 9 |
| Nafarelin | " | 10 |
| Histrelin | " | 9 |
| Calcitonin | Morbus Paget, Osteoporose | 32 |
| Elcatonin | " | 32 |
| Parathormon-(1-34) | Hypocalcämie | 34 |
| Sincalid | Diagnostikum für die Pancreas-Funktion | 8 |
| Ceruletid | " | 10 |
| Pentagastrin | Diagnostikum für die Magenfunktion | 5 |
| Desglugastrin | " | 7 |
| Ociltide | Darm-kontrahierend | 5 |
| Angiogenin | Gefäßneubildung | 123 |
| TGF-beta | Tumortherapie, Immunsuppression | 224 |
| Hirudine | Gerinnungshemmung | 64-65 |

Diese Peptide und Proteine können nach allgemein bekannten Verfahren, z.B. durch Merrifield-Synthese, Gen Engineering und durch Isolierung natürlich vorkommender Peptide und Proteine, erhalten werden.

Die Erfindung betrifft ferner pharmazeutische Zubereitungen, enthaltend eine pharmakologisch wirksame Menge

a) eines, zweier oder dreier Peptide oder Proteine, jeweils bestehend aus 3 bis 225 Aminosäuren, insbesondere aus 3 bis 51 Aminosäuren, oder deren physiologisch verträgliche Salze und

b) eines Hilfsstoffes der allgemeinen Formel I

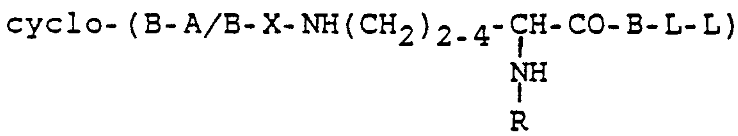 (I)

worin

| | |
|---|---|
| B | Lysin, Ornithin, Histidin, 2,4-Diaminobuttersäure oder Arginin, |
| A/B | Lysin, Ornithin, Histidin, 2,4-Diaminobuttersäure, Arginin, Asparaginsäure oder Glutaminsäure, |
| X | Asparagin, Glutamin, Serin oder Threonin, |
| L | Leucin, Isoleucin, Valin, Threonin, Phenylalanin oder Tryptophan und |
| R | Wasserstoff oder ein Acylrest mit folgenden Strukturen |

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

wobei die Aminosäuren jeweils in ihrer D- oder L-Form vorliegen können, bedeuten.

Ac steht hier für

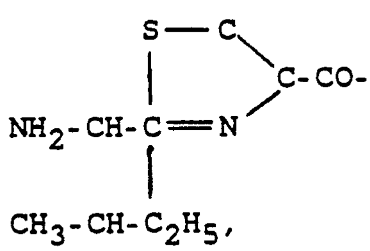

wobei S auch als Sulfoxid oder Sulfon vorliegen und die Doppelbindung hydriert sein kann,

$CH_3-CH_2-CH-(CH_2)_4-CO-$ oder $CH_3-CH-(CH_2)_4-CO-$ .
$(CH_2)_{0-1}-CH_3$ $(CH_2)_{0-1}-CH_3$

Insbesondere sind solche Zubereitungen von Interesse, die einen Hilfsstoff aus der Reihe Bacitracin A, Colistin A oder B, Circulin A oder B, Polymyxin $B_1$ oder $B_2$ oder Polymyxin $D_1$ oder $D_2$ enthalten.

Die erfindungsgemäßen pharmazeutischen Zubereitungen enthalten ferner bevorzugt ein Peptid oder Protein bestehend aus 3 bis 225 Aminosäuren, insbesondere ein Peptid oder Protein mit 3 bis 51 Aminosäuren.

Jedoch auch Zubereitungen, bestehend aus zwei oder drei verschiedenen Peptiden und/oder Proteinen, wie beispielsweise Corticotropin + LHRH + GRH oder Protirelin + LHRH + GRH, in Verbindung mit einem Hilfsstoff wie z.B. Bacitracin sind von Interesse, insbesondere zur Anwendung als Diagnostika.

Die Dosis der Peptide und/oder Proteine und der Hilfsstoffe bei der Anwendung bei Säugern, vorzugsweise beim Menschen, liegen in den erfindungsgemäßen Zubereitungen bzw. Erzeugnissen im Bereich von 10 $\mu$g bis 10 mg pro Peptid/Protein und Anwendung und für den Hilfsstoff bei einer Konzentration von $10^{-5}$ bis $10^{-1}$ mol/l pro Anwendung, bevorzugt zwischen $10^{-4}$ und $10^{-2}$ mol/l.

Die Anwendung der erfindungsgemäßen Zubereitungen kann durch Applikation auf die Schleimhäute, d.h. nasal, buccal, rektal oder vaginal erfolgen. Die nasale Applikation ist hierbei bevorzugt.

Die pharmakologisch verwendbaren Kombinationen der vorliegenden Erfindung und ihre Salze können zur Herstellung von pharmazeutischen Präparaten verwendet werden, welche eine wirksame Menge der Aktivsubstanzen zusammen mit Trägerstoffen enthalten und die sich zur Applikation auf die Schleimhäute eignen, wie beispielsweise Tabletten, Suppositorien, Kapseln, Gele, Filme, Emulsionen, Suspensionen, Aerosole, Lösungen oder Sprays (Sucker, Fuchs, Speiser, Pharmazeutische Technologie, Georg Thieme Verlag 1978).

Vorzugsweise verwendet werden:

1. Wäßrige bzw. wäßrig-alkoholische Lösungen zur Applikation mit einer Tropfpipette bzw. einer Kunststoffquetschflasche oder zur Vernebelung mit einer Dosierzerstäuberpumpe

Die Zubereitung kann neben dem Wirkstoff und dem Resorptionsförderer einen isotonisierenden Zusatz, z.B. Natriumchlorid, Kaliumnitrat, Kalium-Natrium-Phosphat, Polyalkohole wie z.B. Glucose, Mannit, Sorbit, Puffersubstanzen wie beispielsweise Kalium-Natrium-Phosphat, Citronensäure und ihre Salze sowie Mischungen der beiden um einen pH-Bereich von 3 bis 8 einzustellen, ein Konservierungsmittel, z.B. Benzalkoniumchlorid, Benzylalkohol, 1,1,1-Trichlor-2-methyl-2-propanol, Methyl-4 hydroxybenzoat, einen Chelatbildner, z.B. Natrium-EDTA und als Lösungsmittel Wasser oder Mischungen von Wasser mit ($C_1$-$C_4$)-Alkanolen enthalten. Die Lösung wird mit einem geeigneten Gerät appliziert bzw. in die Nase oder auf die Mundschleimhaut gesprüht.

2. Wäßrige bzw. wäßrig-alkoholische Gele zum Einbringen in Körperhöhlen (Mund, Nase, Rektum, Vagina)

Zusätzlich zu 1. enthält ein Gel einen die Viskosität erhöhenden Zusatz, z.B. ein Polyacrylatpolymer oder einen Celluloseether wie z.B. Hydroxypropylmethylcellulose (HPMC), Hydroxyethylcellulose (HEC), Methylhydroxyethylcellulose (MHEC).

3. Suspensionen in Treibgasen

Die Zubereitung kann neben dem mikronisierten Wirkstoff und dem mikronisierten Resorptionsförderer einen Fluorkohlenwasserstoff, z.B. ®Frigen F 113, und ein Suspendierhilfsmittel, z.B. Sorbitantrioleat, enthalten.

Als Treibgase eignen sich Fluorkohlenwasserstoffe, z.B. ®Frigen F 12 und ®Frigen F 114 bzw. ihre Gemische. Die Abfüllung erfolgt in an sich bekannter Weise nach dem Kälteabfüllverfahren oder aber durch Druckfüllung.

4. Verreibungen mit Trägerhilfsstoffen in Kapseln zur intranasalen bzw. inhalativen Anwendung

Die mikronisierten Stoffe (Wirkstoff und Resorptionsförderer) werden gegebenenfalls nach Zugabe eines Mittels zur Verbesserung der Fließeigenschaften, wie z.B. Lactose, in Hartgelatinekapseln abgefüllt. Der Inhalt einer Kapsel wird mit einer Inhalationshilfe, die es erlaubt, das Pulver in einen inhalierbaren Rauch zu überführen, intranasal bzw. pulmonal appliziert.

5. Buccalformen

Wirkstoff und Resorptionsförderer können in gelöster oder suspendierter Form vorliegen. Als Arzneiformen eignen sich Komprimate oder Laminate aus Mischungen von Wirkstoff und Absorptionsförderer in Polymeren. Als Polymere kommen Celluloseether (z.B. HPMC, Carboxymethylcellulose (CMC)) oder Polyacrylate in Frage.

Die nachfolgenden Beispiele sollen die vorliegende Erfindung erläutern, ohne die Erfindung auf die stellvertretend genannten Zubereitungen zu beschränken:

**Beispiel 1**

**Nasallösung**

| | |
|---|---|
| Buserelin | 0,15 mg |
| Bacitracin | 1,50 mg |
| Natriumchlorid | 0,80 mg |
| Citronensäure•$H_2O$ | 0,11 mg |
| Natriumcitrat•$2H_2O$ | 0,15 mg |
| Benzalkoniumchlorid | 0,01 mg |
| Dinatrium-EDTA | 0,01 mg |
| Wasser (gereinigt) | ad 0,1000 ml |

**Beispiel 2**

**Gel**

| | |
|---|---|
| [D-Ala$^2$]Somatorelin-(1-29)-amid | 0,020 mg |
| Colistin | 1,200 mg |
| Polyacrylsäure 940 | 0,400 mg |
| Natriumhydroxid Lösung 15 % | 0,900 mg |
| Glycerin | 15,000 mg |
| Methyl-4-hydroxybenzoat | 0,150 mg |
| Gereinigtes Wasser | ad 100,000 mg |

**Beispiel 3**

**Suppositorium**

| | |
|---|---|
| Salmon-Calcitonin | 0,200 mg |
| Polymyxin | 1,000 mg |
| Suppositorienmasse (Hartfett) | ad 2,500 g |

**Beispiel 4**

**Diagnostikum**

| | |
|---|---|
| Protirelin | 0,050 mg |
| Gonadoliberin | 0,025 mg |
| Somatoliberin | 0,025 mg |
| Bacitracin | 0,250 mg |
| Citronensäure•$H_2O$ | 0,170 mg |
| Dinatriummonohydrogenphosphat•$12H_2O$ | 1,100 mg |
| Natriumchlorid | 0,600 mg |

Benzylalkohol 1,000 mg
Gereinigtes Wasser ad 0,100 ml

**Patentansprüche**

**Patentansprüche für folgende Vertragsstaaten : DE, GB, FR, IT, NL, SE, CH, BE, AT, LU**

1. Verwendung eines Hilfsstoffes der allgemeinen Formel I

$$\text{cyclo-}(B\text{-}A/B\text{-}X\text{-}NH(CH_2)_{2\text{-}4}\text{-}\underset{\substack{|\\ NH\\ |\\ R}}{CH}\text{-}CO\text{-}B\text{-}L\text{-}L) \qquad (I)$$

worin

| | |
|---|---|
| B | Lysin, Ornithin, Histidin, 2,4-Diaminobuttersäure oder Arginin, |
| A/B | Lysin, Ornithin, Histidin, 2,4-Diaminobuttersäure, Arginin, Asparaginsäure oder Glutaminsäure, |
| X | Asparagin, Glutamin, Serin oder Threonin, |
| L | Leucin, Isoleucin, Valin, Threonin, Phenylalanin oder Tryptophan und |
| R | Wasserstoff oder ein Acylrest mit folgenden Strukturen |

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

wobei die Aminosäuren jeweils in der D- oder L-Form vorliegen können,
Ac hier für

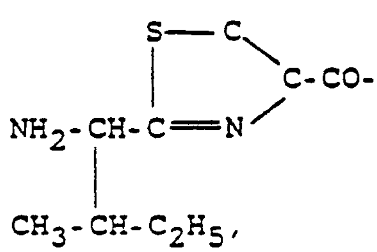

wobei S auch als Sulfoxid oder Sulfon vorliegen und die Doppelbindung hydriert sein kann,

$$CH_3\text{-}CH_2\text{-}\underset{\displaystyle (CH_2)_{0\text{-}1}\text{-}CH_3}{\underset{|}{CH}}\text{-}(CH_2)_4\text{-}CO\text{-} \quad \text{oder} \quad CH_3\text{-}\underset{\displaystyle (CH_2)_{0\text{-}1}\text{-}CH_3}{\underset{|}{CH}}\text{-}(CH_2)_4\text{-}CO\text{-} \quad ,$$

steht, bedeuten oder dessen physiologisch verträglichem Salz zur Herstellung einer pharmazeutischen Zubereitung zur Förderung der Resorption von Peptiden und Proteinen bei Applikation auf die Schleimhäute.

2. Verwendung gemäß Anspruch 1, dadurch gekennzeichnet, daß Bacitracin A, Colistin A oder B, Circulin A oder B, Polymyxin B1 oder B2 oder Polymyxin D1 oder D2 verwendet werden.

3. Pharmazeutische Zubereitung, enthaltend eine pharmakologisch wirksame Menge
   a) eines, zweier oder dreier Peptide oder Proteine, jeweils bestehend aus 3 bis 225 Aminosäuren oder deren physiologisch verträgliche Salze und
   b) eines Hilfsstoffes der allgemeinen Formel I

$$\text{cyclo-}(B\text{-}A/B\text{-}X\text{-}NH(CH_2)_{2\text{-}4}\text{-}\underset{\displaystyle \underset{\displaystyle R}{\underset{|}{NH}}}{\underset{|}{CH}}\text{-}CO\text{-}B\text{-}L\text{-}L) \qquad (I)$$

worin

| | |
|---|---|
| B | Lysin, Ornithin, Histidin, 2,4-Diaminobuttersäure oder Arginin, |
| A/B | Lysin, Ornithin, Histidin, 2,4-Diaminobuttersäure, Arginin, Asparaginsäure oder Glutaminsäure, |
| X | Asparagin, Glutamin, Serin oder Threonin, |
| L | Leucin, Isoleucin, Valin, Threonin, Phenylalanin oder Tryptophan und |
| R | Wasserstoff oder ein Acylrest mit folgenden Strukturen |

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

wobei die Aminosäuren jeweils in ihrer D- oder L-Form vorliegen können,
Ac hier für

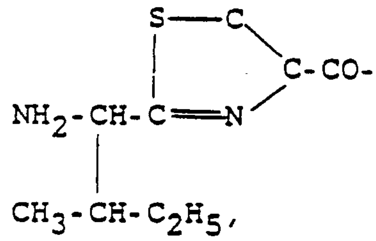

wobei S auch als Sulfoxid oder Sulfon vorliegen und die Doppelbindung hydriert sein kann,

$$CH_3-CH_2-\underset{|}{C}H-(CH_2)_4-CO- \quad \text{oder} \quad CH_3-\underset{|}{C}H-(CH_2)_4-CO-$$
$$(CH_2)_{0-1}CH_3 \qquad\qquad (CH_2)_{0-1}-CH_3$$

steht, bedeuten.

4. Zubereitung gemäß Anspruch 3, dadurch gekennzeichnet, daß ein Hilfsstoff aus der Reihe Bacitracin A, Colistin A oder B, Circulin A oder B, Polymyxin B1 oder B2 oder Polymyxin D1 oder D2 verwendet wird.

5. Zubereitung gemäß einem oder mehrerer der Ansprüche 3 und 4, dadurch gekennzeichnet, daß ein Peptid oder Protein bestehend aus 3 bis 225 Aminosäuren verwendet wird.

6. Zubereitung gemäß einem oder mehrerer der Ansprüche 3 bis 5, dadurch gekennzeichnet, daß ein Peptid oder Protein bestehend aus 3 bis 51 Aminosäuren verwendet wird.

7. Verfahren zur Herstellung einer Zubereitung gemäß einem mehrerer der Ansprüche 3 bis 6, dadurch gekennzeichnet, daß man

   a) ein, zwei oder drei Peptide oder Proteine jeweils bestehend aus 3 bis 225 Aminosäuren der deren Salze und
   b) einen Hilfsstoff der Formel I oder dessen Salz zusammen mit physiologisch annehmbaren Trägern und gegebenenfalls weiteren Hilfs- oder Zusatzstoffen in eine geeignete Darreichungsform bringt.

**Patentansprüche für folgende Vertragsstaaten : ES, GR**

1. Verwendung eines Hilfsstoffes der allgemeinen Formel I

$$cyclo-(B-A/B-X-NH(CH_2)_{2-4}-\underset{\underset{\displaystyle R}{|}}{\underset{\displaystyle NH}{|}}{CH}-CO-B-L-L) \qquad (I)$$

worin

| | |
|---|---|
| B | Lysin, Ornithin, Histidin, 2,4-Diaminobuttersäure oder Arginin, |
| A/B | Lysin, Ornithin, Histidin, 2,4-Diaminobuttersäure, Arginin, Asparaginsäure oder Glutaminsäure, |
| X | Asparagin, Glutamin, Serin oder Threonin, |
| L | Leucin, Isoleucin, Valin, Threonin, Phenylalanin oder Tryptophan und |
| R | Wasserstoff oder ein Acylrest mit folgenden Strukturen |

H-Dab-,
H-Ser-,
H-Ile-,
H-Thr-Dab-,

H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

wobei die Aminosäuren jeweils in der D- oder L-Form vorliegen können,
Ac hier für

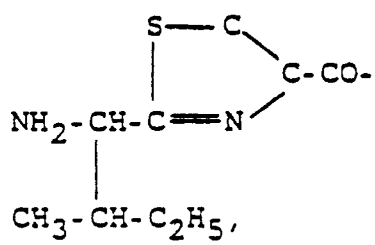

wobei S auch als Sulfoxid oder Sulfon vorliegen und die Doppelbindung hydriert sein kann,

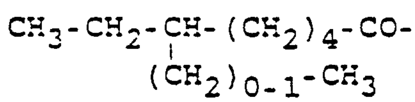 oder 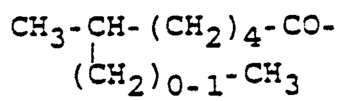 ,

steht, bedeuten oder dessen physiologisch verträglichem Salz zur Herstellung einer pharmazeutischen Zubereitung zur Förderung der Resorption von Peptiden und Proteinen bei Applikation auf die Schleimhäute.

2. Verwendung gemäß Anspruch 1, dadurch gekennzeichnet, daß Bacitracin A, Colistin A oder B, Circulin A oder B, Polymyxin B1 oder B2 oder Polymyxin D1 oder D2 verwendet werden.

3. Verfahren zur Herstellung einer Zubereitung gemäß einem mehrerer der Ansprüche 1 und 2, dadurch gekennzeichnet, daß man

a) ein, zwei oder drei Peptide oder Proteine jeweils bestehend aus 3 bis 225 Aminosäuren der deren Salze und
b) einen Hilfsstoff der Formel I oder dessen Salz zusammen mit physiologisch annehmbaren Trägern und gegebenenfalls weiteren Hilfs- oder Zusatzstoffen in eine geeignete Darreichungsform bringt.

**Claims**
**Claims for the following Contracting States : DE, GB, FR, IT, NL, SE, CH, BE, AT, LU**

**1.** The use of an aid of the formula I

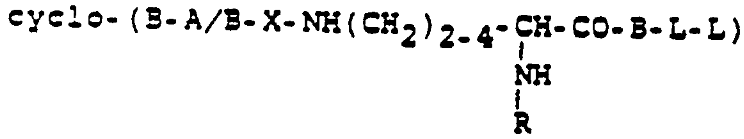 (I)

in which

- B denotes lysine, ornithine, histidine, 2,4-diaminobutyric acid or arginine,
- A/B denotes lysine, ornithine, histidine, 2,4-diaminobutyric acid, arginine, aspartic acid or glutamic acid,
- X denotes asparagine, glutamine, serine or threonine,
- L denotes leucine, isoleucine, valine, threonine, phenylalanine or tryptophan, and
- R denotes hydrogen or an acyl radical having the following structures

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,

Ac-Dab-Thr-Ser-,
Ac-Leu-Glu-Ile-,

it being possible for each of the amino acids to be in the D or L form, and Ac represents here

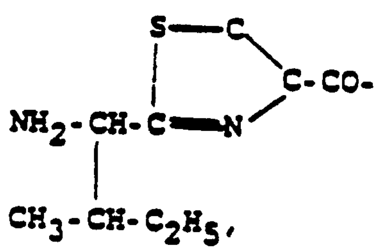

it being possible for S also to be in the form of the sulfoxide or sulfone, and for the double bond to be hydrogenated,

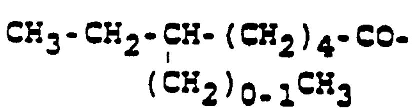 or 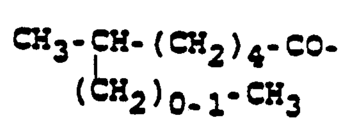

or the physiologically tolerated salt thereof for preparing a pharmaceutical composition for promoting the absorption of peptides and proteins on administration onto the mucosa.

2. The use as claimed in claim 1, wherein bacitracin A, colistin A or B, circulin A or B, polymyxin $B_1$ or $B_2$, or polymyxin $D_1$ or $D_2$ are used.

3. A pharmaceutical composition containing a pharmacologically effective amount
   a) of one, two or three peptides or proteins, each composed of 3 to 225 amino acids, or the physiologically tolerated salts thereof, and
   b) of an aid of the formula I

$$\text{cyclo-[B-A/B-X-NH(CH}_2)_{2-4}\text{-}\underset{\substack{|\\ NH\\ |\\ R}}{\text{CH}}\text{-CO-B-L-L]} \qquad (I)$$

in which

| | |
|---|---|
| B | denotes lysine, ornithine, histidine, 2,4-diaminobutyric acid or arginine, |
| A/B | denotes lysine, ornithine, histidine, 2,4-diaminobutyric acid, arginine, aspartic acid or glutamic acid, |
| X | denotes asparagine, glutamine, serine or threonine, |
| L | denotes leucine, isoleucine, valine, threonine, phenylalanine or tryptophan, and |
| R | denotes hydrogen or an acyl radical having the following structures |

**H-Dab-,**
**H-Ser-,**
**H-Ile-,**
**H-Thr-Dab-,**
**H-Thr-Ser-,**
**H-Dab-Thr-Dab-,**
**H-Dab-Thr-Ser-,**
**H-Leu-Glu-Ile-,**
**Ac-Dab-Thr-Dab-,**
**Ac-Dab-Thr-Ser-,**
**Ac-Leu-Glu-Ile-,**

it being possible for each of the amino acids to be in the D or L form, and Ac represents here

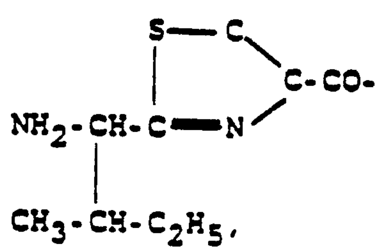

it being possible for S also to be in the form of the sulfoxide or sulfone, and for the double bond to be hydrogenated,

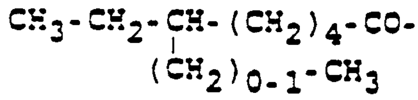 or 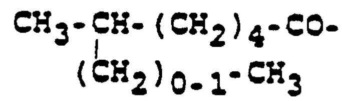

4. A composition as claimed in claim 3, wherein an aid selected from the group comprising bacitracin A, colistin A or B, circulin A or B, polymyxin $B_1$ or $B_2$, or polymyxin $D_1$ or $D_2$ is used.

5. A composition as claimed in one or more of claims 3 and 4, wherein a peptide or protein composed of 3 to 225 amino acids is used.

6. A composition as claimed in one or more of claims 3 to 5, wherein a peptide or protein composed of 3 to 51 amino acids is used.

7. A process for the preparation of a composition as claimed in one or more of claims 3 to 6, which comprises
   a) one, two or three peptides or proteins, each composed of 3 to 225 amino acids, or the salts thereof, and
   b) an aid of the formula I, or the salt thereof, being converted together with physiologically acceptable vehicles and, where appropriate, further aids or additives into a suitable dosage form.

**Claims for the following Contracting States : ES, GR**

1. The use of an aid of the formula I

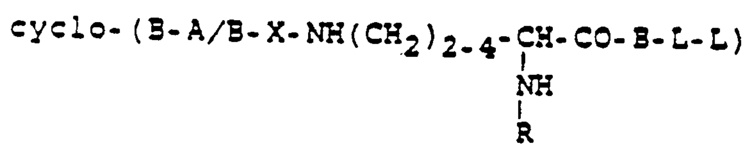 (I)

in which

| | |
|---|---|
| B | denotes lysine, ornithine, histidine, 2,4-diaminobutyric acid or arginine, |
| A/B | denotes lysine, ornithine, histidine, 2,4-diaminobutyric acid, arginine, aspartic acid or glutamic acid, |
| X | denotes asparagine, glutamine, serine or threonine, |
| L | denotes leucine, isoleucine, valine, threonine, phenylalanine or tryptophan, and |
| R | denotes hydrogen or an acyl radical having the following structures |

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,

**Ac-Leu-Glu-Ile-,**

it being possible for each of the amino acids to be in the D or L form, and Ac represents here

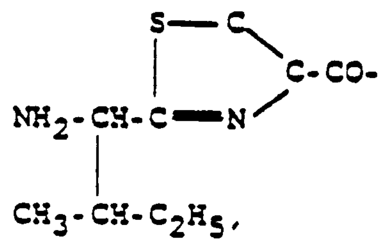

it being possible for S also to be in the form of the sulfoxide or sulfone, and for the double bond to be hydrogenated,

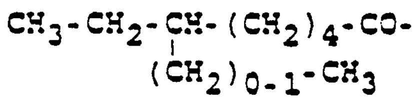 or 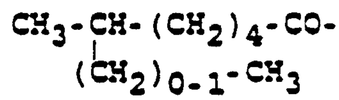

or the physiologically tolerated salt thereof for preparing a pharmaceutical composition for promoting the absorption of peptides and proteins on administration onto the mucosa.

2. The use as claimed in claim 1, wherein bacitracin A, colistin A or B, circulin A or B, polymyxin $B_1$ or $B_2$, or polymyxin $D_1$ or D2 are used.

3. A process for the preparation of a composition as claimed in one or more of claims 1 and 2, which comprises
   a) one, two or three peptides or proteins, each composed of 3 to 225 amino acids, or the salts thereof,
   b) an aid of the formula I, or the salt thereof, being converted together with physiologically acceptable vehicles and, where appropriate, further aids or additives into a suitable dosage form.

**Revendications**
**Revendications pour les Etats contractants suivants : DE, GB, FR, IT, NL, SE, CH, BE, AT, LU**

1. Utilisation d'un adjuvant de formule générale I

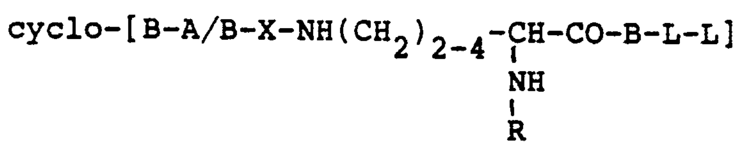 (I)

dans laquelle

| | |
|---|---|
| B | représente la lysine, l'ornithine, l'histidine, l'acide 2,4-diaminobutyrique ou l'arginine, |
| A/B | représente la lysine, l'ornithine, l'histidine, l'acide 2,4-diaminobutyrique, l'arginine, l'acide aspartique ou l'acide glutamique, |
| X | représente l'asparagine, la glutamine, la sérine ou la thréonine, |
| L | représente la leucine, l'isoleucine, la valine, la thréonine, la phénylalanine ou le tryptophane, et |
| R | représente un atome d'hydrogène ou un reste acyle ayant les structures suivantes |

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

les aminoacides pouvant se trouver chacun sous la forme D ou la forme L,
Ac représente

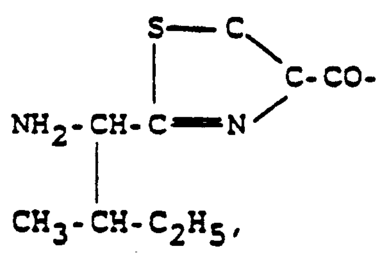

S pouvant également se trouver sous forme de sulfoxyde ou de sulfone, et la double liaison pouvant être hydrogénée,

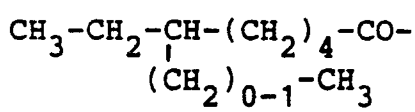 ou 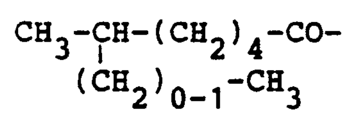

ou d'un sel physiologiquement acceptable de celui-ci, dans la préparation d'une composition pharmaceutique, pour l'accélération de la résorption de peptides et de protéines lors de l'application sur les muqueuses.

2. Utilisation selon la revendication 1, caractérisée en ce que l'on utilise la bacitracine A, la colistine A ou B, la circuline A ou B, la polymyxine $B_1$ ou $B_2$ ou la polymyxine $D_1$ ou $D_2$.

3. Composition pharmaceutique contenant une quantité pharmacologiquement efficace
   a) de 1, 2 ou 3 peptides ou protéines, constitués chacun de 3 à 225 aminoacides, ou de sels physiologiquement acceptables de ceux-ci, et
   b) d'un adjuvant de formule générale I

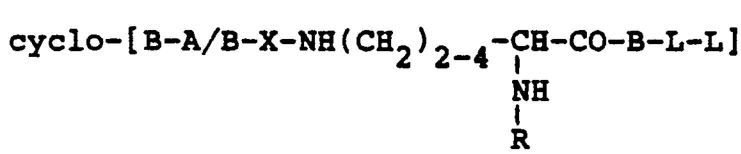 (I)

dans laquelle

- B représente la lysine, l'ornithine, l'histidine, l'acide 2,4-diaminobutyrique ou l'arginine,
- A/B représente la lysine, l'ornithine, l'histidine, l'acide 2,4-diaminobutyrique, l'arginine, l'acide aspartique ou l'acide glutamique,
- X représente l'asparagine, la glutamine, la sérine ou la thréonine,
- L représente la leucine, l'isoleucine, la valine, la thréonine, la phénylalanine ou le tryptophane, et
- R représente un atome d'hydrogène ou un reste acyle ayant les structures suivantes

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

les aminoacides pouvant se trouver chacun sous la forme D ou la forme L,
Ac représente

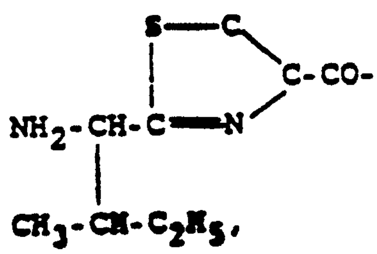

S pouvant également se trouver sous forme de sulfoxyde ou de sulfone, et la double liaison pouvant être hydrogénée,

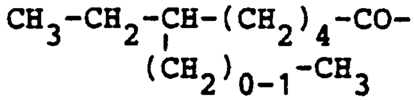 ou 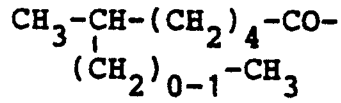

4. Composition selon la revendication 3, caractérisée en ce que l'on utilise un adjuvant choisi parmi la bacitracine A, la colistine A ou B, la circuline A ou B, la polymyxine $B_1$ ou $B_2$, ou la polymyxine $D_1$ ou $D_2$.

5. Composition selon une ou plusieurs des revendications 3 et 4, caractérisée en ce que l'on utilise un peptide ou une protéine constitués de 3 à 225 aminoacides.

6. Composition selon une ou plusieurs des revendications 3 à 5, caractérisée en ce que l'on utilise un peptide ou une protéine constitués de 3 à 51 aminoacides.

7. Procédé pour la préparation d'une composition selon une ou plusieurs des revendications 3 à 6, caractérisé en ce que l'on met sous une forme pharmaceutique appropriée
   a) 1, 2 ou 3 peptides ou protéines constitués chacun de 3 à 225 aminoacides, ou leurs sels, et
   b) un adjuvant de formule I ou un sel de celui-ci, conjointement avec des véhicules physiologiquement acceptables et éventuellement d'autres adjuvants ou additifs.

**Revendications pour les Etats contractants suivants : ES, GR**

1. Utilisation d'un adjuvant de formule générale I

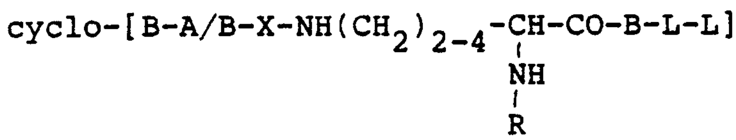

(I)

dans laquelle

- B représente la lysine, l'ornithine, l'histidine, l'acide 2,4-diaminobutyrique ou l'arginine,
- A/B représente la lysine, l'ornithine, l'histidine, l'acide 2,4-diaminobutyrique, l'arginine, l'acide aspartique ou l'acide glutamique,
- X représente l'asparagine, la glutamine, la sérine ou la thréonine,
- L représente la leucine, l'isoleucine, la valine, la thréonine, la phénylalanine ou le tryptophane, et
- R représente un atome d'hydrogène ou un reste acyle ayant les structures suivantes

H-Dab-,
H-Ser-,
H-Ile-,
H-Thr-Dab-,
H-Thr-Ser-,
H-Dab-Thr-Dab-,
H-Dab-Thr-Ser-,
H-Leu-Glu-Ile-,
Ac-Dab-Thr-Dab-,
Ac-Dab-Thr-Ser-,
Ac-Leu-Glu-Ile-,

les aminoacides pouvant se trouver chacun sous la forme D ou la forme L,
Ac représente

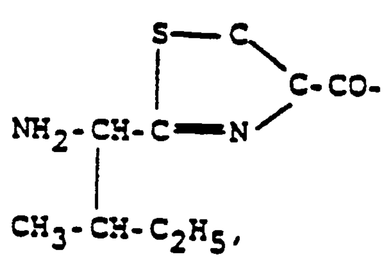

S pouvant également se trouver sous forme de sulfoxyde ou de sulfone, et la double liaison pouvant être hydrogénée,

$$CH_3-CH_2-\underset{|}{C}H-(CH_2)_4-CO-$$
$$(CH_2)_{0-1}-CH_3$$

ou

$$CH_3-\underset{|}{C}H-(CH_2)_4-CO-$$
$$(CH_2)_{0-1}-CH_3$$

ou d'un sel physiologiquement acceptable de celui-ci, dans la préparation d'une composition pharmaceutique, pour l'accélération de la résorption de peptides et de protéines lors de l'application sur les muqueuses.

2. Utilisation selon la revendication 1, caractérisée en ce que l'on utilise la bacitracine A, la colistine A ou B, la circuline A ou B, la polymyxine $B_1$ ou $B_2$ ou la polymyxine $D_1$ ou $D_2$.

3. Procédé pour la préparation d'une composition selon une ou plusieurs des revendications 1 et 2, caractérisé en ce que l'on met sous une forme pharmaceutique appropriée
   a) 1, 2 ou 3 peptides ou protéines constitués chacun de 3 à 225 aminoacides, ou leurs sels, et
   b) un adjuvant de formule I ou un sel de celui-ci, conjointement avec des véhicules physiologiquement acceptables et éventuellement d'autres adjuvants ou additifs.